# EUROPÄISCHE PATENTSCHRIFT

(11) **EP 0 687 443 B1**
(45) Veröffentlichungstag und Bekanntmachung des Hinweises auf die Patenterteilung: **15.12.1999**
(21) Anmeldenummer: 95107539.9
(22) Anmeldetag: 18.05.1995
(51) Int. Cl.: A61B 6/04, A61B 6/02

(54) **Vorrichtung zur Positionierung und Markierung eines Patienten an Diagnosegeräten vor und nach der Durchleuchtung in einem Computertomographen**
Means for positioning and marking of a patient on diagnostic equipments before and after x-ray examination in a computer tomograph
Dispositif de positionnement et repérage d'un patient pour les appareils diagnostiques avant et après l'examen dans un appareil tomographie

(30) Priorität: 17.06.1994 DE 4421315
(43) Veröffentlichungstag der Anmeldung: 20.12.1995
(73) Patentinhaber: LAP GmbH Laser Applikationen, D-21337 Lüneburg (DE)
(72) Erfinder: Röckseisen, Armin, Dr., D-21379 Scharnebeck (DE)
(74) Vertreter: Patentanwälte Hauck, Graalfs, Wehnert, Döring, Siemons

(56) Entgegenhaltungen:
- EP-A- 0 480 035
- US-A- 4 242 587
- US-A- 4 442 533
- US-A- 4 629 989

## Beschreibung

Die Erfindung bezieht sich auf eine Vorrichtung zur Positionierung und Markierung und Markierung eines Patienten an Diagnosegeräten vor oder nach der Durchleuchtung in einem Computertomographen nach dem Oberbegriffs des Patentanspruchs 1.

In der Strahlentherapie ist es notwendig, den Strahl der Bestrahlungsquelle präzise auf das zu bestrahlende Gebiet eines Patientenkörpers auszurichten, und zwar genau reproduzierbar. Zur Verringerung der Belastung nicht zu therapierender Bereiche wird die Strahlungsquelle um ein sogenanntes Isozentrum geschwenkt, so daß im Zentrum des zu bestrahlenden Gebietes stets eine gleiche Dosisleistung erzielt wird, im benachbarten nicht zu behandelnden Gebiet hingegen eine deutlich verringerte Belastung stattfindet. Ein Patient ist daher im Hinblick auf das Bestrahlungsgerät so auszurichten, daß das Zentrum des zu bestrahlenden Gebietes mit dem Isozentrum des Bestrahlungsgerätes zusammenfällt. Die Lage des zu bestrahlenden Gebietes kann durch geeignete Diagnosemethoden, beispielsweise einem Computertomographen (CT) ermittelt werden. Dabei sind nicht nur die Koordinaten des Zentrums des zu bestrahlenden Gebietes wesentlich, sondern auch die Ausdehnung des Gebietes sowie der Umfang, der vom Bestrahlungsgerät erfaßt werden soll. Der Fokus des Bestrahlungsgebietes liegt üblicherweise zwischen Strahlungsquelle und dem Körper des Patienten. Dadurch ergibt sich eine divergente Strahlung, die je nach den Abmessungen des Körpers des Patienten eine mehr oder weniger große Fläche auf der Haut abdeckt. Mit Hilfe einer entsprechenden Maskierung kann erreicht werden, daß diese Fläche verkleinert wird.

Zur entsprechenden Ausrichtung des Patienten im Hinblick auf das Bestrahlungsgerät ist es notwendig, die Lage des zu bestrahlenden Gebietes anzuzeigen bzw. zu markieren. Es ist bekannt, mit Hilfe von in einem Bestrahlungsraum fest angeordneten Linienlasersystemen den Patienten auf einer Liege in eine präzise Lage zu bringen, bevor er in das Bestrahlungsgerät gefahren wird.

Die Koordinaten des zu bestrahlenden Gebietes werden mit Hilfe zum Beispiel eines Computertomographen ermittelt. Die Koordinaten werden auf der Haut des Patientenkörpers so markiert, daß bei einer Ausrichtung des Patienten zum Bestrahlungsgerät das Zentrum des Tumors im Isozentrum des Bestrahlungsgerätes liegt.

Aus EP-A-0 480 035 ist eine Vorrichtung bekanntgeworden, bei der oberhalb eines auf einer Liege liegenden Patienten ein erster Projektor und seitlich neben der Liege ein zweiter und ein dritter Projektor angeordnet sind. Der erste Projektor ist quer zur Körperachse horizontal verstellbar. Die beiden anderen Projektoren sind in der Höhe verstellbar. Alle Projektoren erzeugen ein Lichtkreuz auf dem Patientenkörper und werden mit Hilfe numerisch gesteuerter Antriebe bewegt. Die Steuerung erfolgt über eine Positioniersteuervorrichtung, welche aus vom Computertomographen ermittelten Daten die Position bestimmt, in welche die Markierungen gefahren werden. Die Markierungen zeigen ein zu bestrahlendes Gebiet an, das zuvor im Computertomographen ermittelt und im Hinblick auf die Koordinaten bestimmt wurde.

Der Erfindung liegt die Aufgabe zugrunde, eine Vorrichtung zur Positionierung und Markierung eines Patienten mit Hilfe eines Computertomographen zu schaffen.

Diese Aufgabe wird durch die Merkmale des Patentanspruchs 1 gelöst.

Die erfindungsgemäße Vorrichtung sieht mindestens vier Linienlaser vor, die entlang einer Achse verfahrbar in einem Raum angeordnet sind. Mindestens zwei oberhalb des Patienten angeordnete Lasergeräte erzeugen eine sogenannte Sagittallinie und eine sogenannte Transversallinie. Vorzugsweise werden zwei Linienlaser für die Erzeugung einer gemeinsamen Transversallinie verwendet, die annähernd um den Körper herumläuft. Durch die Verfahrbarkeit der Linienlaser kann zum Beispiel die Transversallinie in der Sagittalachse verfahren werden, während die Sagittallinie in der Transversalachse bewegt werden kann. Zwei weitere Lasergeräte projizieren je eine Linie in der Körperachse seitlich auf den Körper. Diese können synchron senkrecht zu den erwähnten Achsen auf und ab bewegt werden. Die erwähnten Linien sind Begrenzungen von Lichtebenen, die von den Lasergeräten ausgehen. Die Strahlenebenen schneiden sich in einem Punkt eines orthogonalen Koordinatensystems. Bei der gewünschten Markierung eines zu bestrahlenden Gebietes wird angestrebt, daß der Nullpunkt des Koordinatensystems mit dem Zentrum des zu bestrahlenden Gebietes übereinstimmt, so daß dieses Zentrum mit dem Isozentrum des Bestrahlungsgerätes zur Deckung gebracht werden kann.

Die Positionier-Steuervorrichtung steuert die numerischen Antriebe für die Lasergeräte, um die beschriebene Bewegung herbeizuführen. Das Maß der Bewegung hängt ab von den Steuerdaten, die mit Hilfe eines manuell bedienbaren Eingabegerätes in die Steuervorrichtung eingegeben werden. Diese Daten werden mit Hilfe des Diagnosegerätes, beispielsweise eines Computertomographen ermittelt. Mit Hilfe des Computertomographen können die Koordinaten des Zentrums des zu bestrahlenden Gebietes (Tumorzentrum) ermittelt werden. Es können jedoch auch die Koordinaten für die Ausdehnung des Tumors entlang der drei Achsen ermittelt werden. Sind diese Koordinaten bekannt, können sie über das Eingabegerät in die Steuervorrichtung gegeben werden, und die Steuervorrichtung bewegt die projizierten Linien auf die gewünschte Position. Zusätzlich zu den Koordinaten des Tumors können auch weitere Daten eingegeben werden, nämlich die Divergenz des Bestrahlungsgerätes Sie bestimmt die bestrahlte Hautfläche bzw. die bestrahlte Fläche des Tumors. Diese Fläche ist aber auch abhängig von dem Abstand zwischen Haut und Fokus des Bestrahlungsgerätes. Durch die Verstellung der Linien in die gewünschten Positionen kann daher nicht nur das Isozentrum für die Bestrahlung ermittelt werden, sondern auch zum Beispiel die senkrechte Projektion der geometrischen Ausdehnung des Tumors auf der Hautoberfläche in den drei Achsen. Ferner lassen sich die Eintrittsfeldgrenzen im Fokus-Haut-Abstand ermitteln und markieren sowie auch beliebige andere Positionen nach individueller Koordinateneingabe.

Durch die Verwendung eines separaten Eingabegerätes ist die erfindungsgemäße Vorrichtung vollkommen unabhängig von der jeweiligen Ausführung des Computertomographen oder eines vergleichbaren Diagnosegerätes.

Die Erfindung wird nachfolgend anhand von Zeichnungen näher erläutert.
- Fig. 1: zeigt sehr schematisch einen Teil der Vorrichtung nach der Erfindung.
- Fig. 2: zeigt schematisch die Bedieneinheit für eine Steuervorrichtung für die Vorrichtung nach der Erfindung.

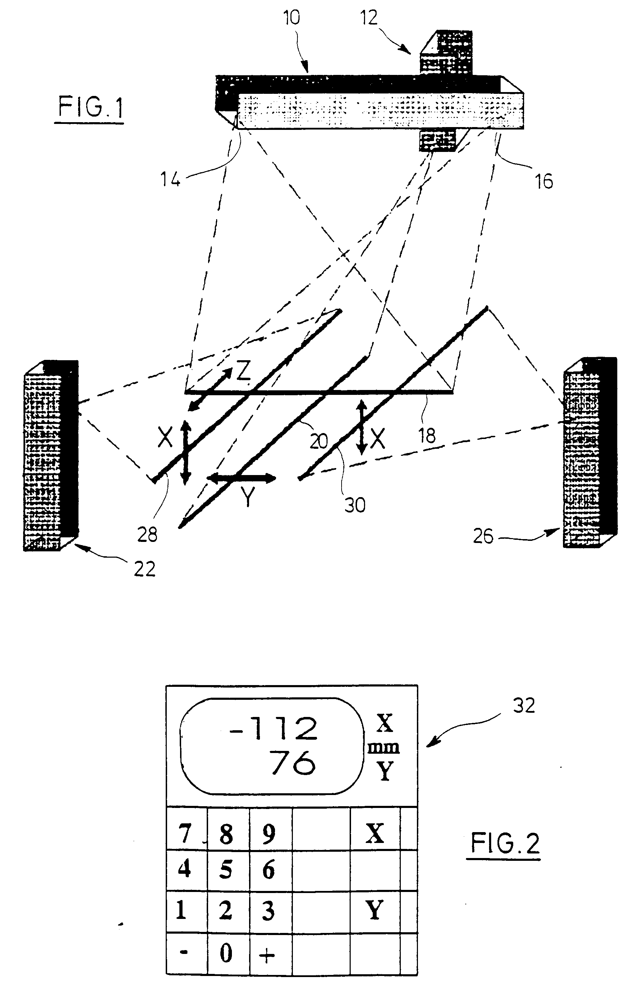

In Fig. 1 sind zwei Lasergeräte 10, 12 angedeutet, die an der Decke eines Raumes angebracht werden, in dem sich auch ein Computertomograph befindet (nicht gezeigt) sowie eine verfahrbare Liege für den Patienten zum Transport des Patienten in den Computertomographen und aus diesem heraus, wobei die Liege ebenfalls mit Hilfe eines numerisch gesteuerten Ahtriebes verfahrbar ist. Das Lasergerät 10 besteht aus zwei Lasern 14, 16 zur Erzeugung einer gemeinsamen tranversalen Linie 18, die nahezu um den nicht gezeigten Körper des Patienten herumläuft.

Der Laser 12 erzeugt eine Sagittallinie, wodurch der Patient auf der Liege zu dieser Linie ausgerichtet werden kann. Zwei seitliche Lasergeräte 22, 26 projizieren je eine Linie in Körperachse seitlich auf den Körper. Diese sind mit 28 bzw. 30 bezeichnet.

Die Lasergeräte 10, 12, 22 und 26 sind mit Hilfe von numerisch gesteuerten Antrieben (nicht gezeigt) entlang einer Achse verstellbar, und zwar derart, daß die Transversallinie 18 entlang der Sagittalachse verfahrbar ist (Z-Achse), die Sagittallinie 20 entlang der Transversalachse (Y-Achse) und die seitlichen Linien 28, 30 entlang der X-Achse.

Die Ebenen, in denen die erwähnten Linien 18, 20, 28 und 30 liegen, schneiden sich in einem Nullpunkt des Koordinatensystems, das durch die Verfahrbarkeit der Lasergeräte verfahren werden kann, wie auch einzelne Linien verfahren werden können.

Die nicht gezeigten Antriebe werden von einer nicht gezeigten Positionier-Steuervorrichtung gesteuert, die ihrerseits Befehlsdaten von einem Eingabegerät 32 (Fig. 2) erhält, von dem die Eingabetastatur dargestellt ist.

Nachfolgend wird ein Ablauf des Betriebes der Vorrichtungen nach Fig. 1 und 2 erläutert. In der Ausgangsposition steht der Sagittallaser 12 in der Mitte, während die beiden seitlichen Linien 28, 30 in einer definierten Nullstellung stehen. Die Transversallinie 18 verläuft in einem definierten Abstand zur Querachse des nicht gezeigten Computertomographen. Wie schon erwähnt, schneiden sich die Laserlinien in einem virtuellen Isozentrum. Der Patient kann nach diesen Linien ausgerichtet und dem Computertomographen zugeführt werden. Nach der Diagnose ist die Lage und die Ausdehnung des Tumors ermittelt worden. Der Patient wird dann mit Hilfe der Steuerung des Computertomographen auf der Liege so weit herausgefahren, daß das Tumorzentrum in der Ebene der Transversallinie 18 liegt. Die beiden übrigen Koordinaten des Tumorzentrums (X und Y) werden aus dem Ergebnis des Computertomographen manuell in das Eingabegerät 32 eingegeben, wodurch die Laserlinien 20, 28, 30 in die angegebenen Positionen fahren. Das nunmehr auf den Körper des Patienten projizierte Raumkoordinatennetz aus Laserlinien gibt die Lage des Zentrums des Tumors an, das durch Markierung auf den Patienten übertragen werden kann, um eine reproduzierbare Lagerung zum Isozentrum des Bestrahlungsgerätes zu erreichen.

Durch Eingabe weiterer Parameter bzw. Koordinaten können die Linien ferner verfahren werden, um die geometrische Ausdehnung des Tumors auf der Haut darzustellen. Hierbei ist von Interesse zum einen die Ausdehnung des Tumors in der senkrechten Schnittebene des Computertomographen (Projektion der geometrischen Daten auf die Hautoberfläche) und das Eintrittsfeld für die Bestrahlung, das von der Divergenz des Bestrahlungsgerätes und dem Abstand zwischen dem Fokus und der Haut bestimmt wird. Aus den eingegebenen Daten für diese Koordinaten und den Parametern des Bestrahlungsgerätes (Divergenz und Abstand) ermittelt der Steuerrechner die anzufahrenden Positionen. Die auf der Haut abgebildeten Linien werden zur weiteren Markierung des Patienten genutzt.

## Patentansprüche

1. Vorrichtung zur Positionierung und Markierung eines Patienten an Diagnosegeräten vor oder nach der Durchleuchtung in einem Computertomographen, mit einem oberhalb des auf einer verfahrbaren Liege gelagerten Patienten angeordneten Projektor, der quer zur Körperachse verstellbar ist, jeweils einem auf jeder Seite des Patienten angeordneten Projektor, die in der Höhe verstellbar sind, wobei die Projektoren mittels numerisch gesteuerter Antriebe angetrieben sind und eine Lichtmarkierung auf dem Körper des Patienten erzeugen, einer Positioniersteuervorrichtung für die Antriebe und einem Eingabegerät für die Positioniersteuervorrichtung zwecks Eingabe von die Lage und die Ausdehnung eines zu bestrahlenden Gebiets betreffenden, vom Computertomographen ermittelten Koordinaten, dadurch gekennzeichnet, daß oberhalb der verfahrbaren Liege zwei Linienlaser (10, 12) gelagert sind, von denen einer eine Sagittallinie (20) und der andere eine Transversallinie (18) auf den Patientenkörper projiziert und die jeweils quer zur erzeugten Linie (18, 20) verstellbar sind und die seitlich angeordneten Projektoren ebenfalls Linienlaser (22, 26) sind, die seitliche Linien (28, 30) auf den Patientenkörper projizieren.

2. Vorrichtung nach Anspruch 1, dadurch gekennzeichnet, daß die Transversallinie (18) von zwei Linienlasern (14, 16) erzeugt wird.

## Claims

1. A device for positioning and marking a patient on diagnosis apparatus before or after the through-illuminating in a computer tomograph, with a projector which is arranged above the patent mounted on a traversable rest and which is adjustable transversely to the body axis, with in each case a projector which is arranged on each side of the patient and are adjustable in height, wherein the projectors are driven by way of numerically controlled drives and produce a light marking on the body of the patient, with a position control device for the drives and with an input apparatus for the position control device for the purpose of inputting co-ordinates which concern the position and extension of an area to be irradiated and which are determined by the computer tomograph, characterised in that above the traversable rest there are mounted two line lasers (10, 12) of which one projects a sagittal line (20) onto the patient and the other a transversal line (18) and which in each case are adjustable transversely to the produced line (18, 20) and the laterally arranged projectors are likewise line lasers (22, 26) which project lateral lines (28, 30) onto the patient body.

2. A device according to claim 1, characterised in that the transversal line (18) is produced by two line lasers (14, 16).

## Revendications

1. Dispositif pour positionner et marquer un patient sur des appareils de diagnostic avant ou après exposition aux rayons dans un tomographe commandé par ordinateur, comportant un projecteur qui est disposé au-dessus du patient étendu sur une couche déplaçable et qui peut être positionné transversalement par rapport à l'axe du corps, des projecteurs qui Dont disposés sur chacun des côtés du patient et qui peuvent être positionnés en hauteur, les projecteurs étant mus par des moyens d'entraînement à commande numérique et assurant un marquage optique sur le corps du patient, un dispositif de commande de positionnement pour les moyens d'entraînement et un appareil d'entrée pour le dispositif de commande de positionnement en vue d'introduire des coordonnées concernant la position et l'étendue d'une zone à soumettre au rayonnement, ces coordonnées étant déterminées par le tomographe commandé par ordinateur,
caractérisé en ce qu'au-dessus de la couche déplacable sont installés deux lasers linéaires (10, 12), dont l'un projette une ligne sagittale (20) et l'autre une ligne transversale (18) sur le corps du patient, et qui sont chacun réglables en position transversalement par rapport à la ligne (18, 20) tracée, et en ce que les projecteurs disposés latéralement sont, de même, des lasers linéaires (22, 26) qui projettent des lignes latérales (28, 30) sur le corps du patient.

2. Dispositif suivant la revendication 1, caractérisé en ce que la ligne transversale (18) est réalisée par deux lasers linéaires (14, 16).
